Europäisches Patentamt

European Patent Office

Office européen des brevets

(19)

(11) Publication number: **0 362 419**

**A1**

(12) # EUROPEAN PATENT APPLICATION

(21) Application number: 88116360.4

(22) Date of filing: 04.10.88

(51) Int. Cl.<sup>5</sup>: **A61K 35/08** , **A61K 7/48** ,
    //(**A61K35/08,35:02**)

(43) Date of publication of application:
    **11.04.90 Bulletin 90/15**

(84) Designated Contracting States:
    **AT BE CH DE ES FR GB GR IT LI LU NL SE**

(71) Applicant: **ENERGIAGAZDALKODASI INTEZET**
    **33-34, Bem-rakpart**
    **H-1027 Budapest II(HU)**

(72) Inventor: **Csermely, Miklos, Dr.**
    **Solyomvölgyi ut 14**
    **H-1029 Budapest(HU)**
    Inventor: **Göde, György, Dr.**
    **Verseny u.21c**
    **H-8360 Keszthely(HU)**
    Inventor: **Halmos, Laszlo, Dr.**
    **Karthauzi u. 6**
    **H-1121 Budapest(HU)**
    Inventor: **Markovics, Jozsef**
    **Lajos u.49/a**
    **H-1036 Budapest(HU)**

Inventor: **Papp, Istvan**
    **Ady Endre u.1**
    **H-1024 Budapest(HU)**
    Inventor: **Puchinger, Ede**
    **Téglagyari u.26**
    **H-8400 Ajka(HU)**
    Inventor: **Szerencsés, Eva, Dr.**
    **Menyecske u. 7**
    **H-1112 Budapest(HU)**
    Inventor: **Toth, Karoly, Dr.**
    **Tapolcsanyi u. 3**
    **H-1022 Budapest(HU)**
    Inventor: **Vamos, György, Dr.**
    **Hadik A. u. 13/B**
    **H-1125 Budapest(HU)**

(74) Representative: **Patentanwälte Viering &**
    **Jentschura**
    **Steinsdorfstrasse 6**
    **D-8000 München 22(DE)**

(54) **Process for the preparation of artificial medicinal mud.**

(57) The present invention relates to the preparation of a suspension, especially medicinal mud. According to the invention 25-200 % by weight of flue-ash (calculated for medicinal water or concentrated medicinal water) formed in power stations, having a particle size of 0.002-1.00 mm, having a CaO content of less than 10 % of CaO and a carbon (coke) content of 5-25 % are suspended in a medicinal water or concentrated medicinal water and the suspension is left to stand for 24-72 hours before use.

If desired the medicinal mud of the invention is prepared in an air-dry state and the moisture content thereof is set before use.

EP 0 362 419 A1

## PROCESS FOR THE PREPARATION OF ARTIFICIAL MEDICINAL MUD

### TECHNICAL FIELD OF THE INVENTION

The present invention relates to the preperation of artificial medicinal mud.

The present invention relates particularly to the preparation of a suspension consisting of a) a liquid traction comprising medicinal water or water comprising ions assuring the medicinal effect of medicinal waters and b) a solid fraction being an inert substance having a particle size of 0.002 to 1.0 mm.

Under the term "medicinal water" the water of such springs is understood which exhibits advantageous physiological effect due to its ion composition, e.g. it can be used for the treatment on diseases of articular organs and locomotion organs. In course of balneotherapeutical treatments not only medicinal water but a mud comprising said water of medicinal springs is also used.

As it is known medicinal muds are substances occuring in the nature in mud-like consistency. Due to their therapeutically active ingredient-content they can directly be used for balneological purposes in natural form as mud-pack.

In the medical literature, medicinal muds are called "peloids" (mud-like). Peloids formed by geological processes are finely dispersed in water. They are used in dispersed form in the therapeutical practice for bathing and wrapping of the body. The characteristic particle size of peloids varies between 0.002 to 1 mm.

It is also known that the wide and increasing use of peloids of natural origin has resulted in the depletion of the stockpile. For this reason there is need to substitute natural peloids, so the meaning of the term "peloid" was to be widened.

Different attempts had been made to find substituting methods to produce artificial medicinal muds.

One of the possibilities resides in that, in order to increase the quantity of the medicinal mud, peat is introduced into the medicinal spring water. The streaming medicinal spring water cuts fine particles - started to get carbonized - from the original mud in the so-called setting lake. The medicinal mud thus formed is a transition substance between peats and spring muds, i.e. it is a mixed mud. Thus the term "peloid" is extended to these mixed muds as well.

The disadvantage of the preparation of said mixed muds is that only a small part of peats is used properly, the main part of it remains unused. Therefore the transportation and placing of the remaining portion of peat causes further difficulties and additional costs.

A further disadvantage of this process is that the organic substance-content of medicinal mud obtained by using peat is often above the permitted value (18 %), therefore it cannot be used for medical treatments.

Due to the continuous decrease of formation and possible exploitation of natural medicinal mud and simultaneously the progressive increase of need for medical bath treatments involving curative mud therapies, the price of medicinal muds increases highly. In order to compensate said high prices of the medicinal muds it became a spread practice that the medicinal muds are repeatedly used up. However, this method is accompanied by a significant increase of costs as the sterilization of the already used mud, i.e. the refreshment and purification of mud loaded with human body-fluids are time- and money-consuming process.

Our aim is to eliminate the above-mentioned drawbacks by preparing a medicinal mud (peloid) the solid component of which is very cheap and widely available.

### SUMMARY OF THE INVENTION

The invention is based on the recognition that flue-ash (flying ashes) formed in power stations can preferably be used for substituting the solid component of peloids of natural origin. The flue-ash formed in said power stations is available in almost unlimited amount in the neighbourhood of power plants kept going with coal, lignite, etc.

Therefore the present invention relates to the preparation of artificial medicinal mud. According to the invention 25-200 % by weight (calculated on the basis of the medicinal water) of flue-ash formed in said power stations are suspended in medicinal water or concentrated medicinal water, and the suspension is left to stand for 24-72 hours before use. The process of the invention is further characterized by eliminating physically bound water from the suspension and producting an air-dry product, preferably having a moisture content of below 5 %.

## DETAILED DESCRIPTION OF THE INVENTION

According to a preferable embodiment of the process of the invention medicinal water and flue-ash formed in power stations are mixed in a weight ratio of 1:1.

Our experiments have proved that those fractions of flue-ash formed in power stations which have a particle size range of 0.002 - 0.800 mm and a CaO content of less than 10 % by weight, further a carbon (coke) content within the range of 5-25 % by weight, are able to absorb and desorb the active ingredients of medicinal waters under suitable conditions.

In addition to the physical adsorption provided by the great specific surface and sponge-like morphology of flue-ash comprising a high number of small pores, chemical adsorption (chemisorption) also occures in smaller extent depending on the carbon (coke) content of flue-ash.

The desorption of the material adsorbed in such a way on the surface of flue-ash (adsorptivum) can be carried out by a moderate heat-treatment, by setting the temperature of the medicinal mud to 40-60 °C. The increase of the temperature of the mud is advantageous during several therapeutic treatments from the point of view of the patient.

The peloids prepared by the process of the invention are suitable for direct and indirect use as well.

In the course of direct use such amount of flue-ash formed in power stations is added to the medicinal water which assures the required consistency for the intended use. After some day, preferably 24-72 hour, standing the mud can be used on site.

In course of indirect use the suspension of medicinal water and flue-ash formed in power stations is dried to an air-dried from, i.e. dried to a moisture content of less than 5 % by weight. The air-dry medicinal mud can easily and cheaply be prepared and transported, put onto the market and used by the patient on his own at home. When the patient uses the mud on his own, it can be used by diluting with any kind of water (depending on the intended use), comprising no medicinal ingredient, e.g. tap-water. In this case the moisture content of medicinal mud suitable for direct use is preferably set to 33-50 % by weight.

Under the term "medicinal water" the mother liquor remaining as distillation residue when the medicinal waters are evaporated, is also understood. In these liquors the concentration of the therapeutically active ingredients can be significantly higher than in the original medicinal water.

In the process according to the invention any inert substance can be used as the solid fraction of the medicinal mud the particle size of which falls within the above-mentioned range. Under the term "inert substance" such substances are understood which do not chemically react with the components of medicinal water and do not exhibit skin-irritating effect when the mud is used.

There are known such flue-ashes formed in power stations which comprises $^{226}$Ra isotope thus the peloids prepared from such flue-ashes formed in power stations are radioactive. The measure of this radioactivity falls within the range of the radioactivity of naturally radioactive medicinal waters, medicinal muds.

The ion composition of medicinal mud prepared by the process of the invention and the composition of liquid fraction of medicinal mud suitable for indirect use were measured and compared to the ion composition of the original medicinal water. This is called the adsorption/desorption effectively of mud. We have found that this value is at least 80 % in the case of mud for direct use, while when e.g. the peloid suitable for indirect use has a moisture content of 33 % or 50 % by weight this value is at least 7 % or 5 %, respectively.

The most important advantages of the peloid prepared by the process of the invention are as follows:

- The medicinal mud prepared from flue-ash formed in power stations has less volumetric weight than peloids of natural origin, thus - if the same volume is used - there is a smaller pressure on the ill part of body.

- The specific preparation cost of medicinal mud prepared from flue-ash formed in power stations is low, therefore the multiple use and need for purification of medicinal muds can be eliminated. By using the medicinal mud prepared from flue-ash formed in power stations the hygienic prescriptions can more easily and securely be complied with.

- Due to the chemical reaction occuring in course of the formation of flue-ash in power stations, from the point of human organism, this substance is pure and free of microorganisms. It can much more favourably used than natural peloids which also comprise organic substances.

- The use of flue-ash formed in power stations - being a waste material - means a good possibility for using a waste which is, from viewpoint of environment protection, advantageous.

- As we have already pointed out, the peloids prepared by the process of the invention are suitable for direct and indirect use.

The invention is illustrated by the following, non-limiting examples.

### Example 1

A medicinal mud suitable for direct use is prepared by the process of the invention.

100 g of fine flue-ash (the parameters of which are listed in Table II) are added to 100 g of mother liquor obtained by distilling the medicinal water of Sárvár. This mother liquor is highly concentrated in respect of some active ingredients of the original medicinal water. The composition of the mother liquor is shown in Table I.

Table I

| Composition of the mother liquor (g/l) | | |
|---|---|---|
| Potassium | $K^+$ | 81.00 |
| Sodium | $Na^+$ | 100.00 |
| Ammonium | $NH_4^+$ | $0.09 . 10^{-3}$ |
| Calcium | $Ca^{2+}$ | $45.8 . 10^{-3}$ |
| Magnesium | $Mg^{2+}$ | $18.3 . 10^{-3}$ |
| Iron | $Fe^{2+}$ | $0.90 . 10^{-3}$ |
| Manganese | $Mn^{2+}$ | less than 0.5 |
| Lithium | $Li^+$ | 2.900 |
| Copper | $Cu^{2+}$ | 0.250 |
| Nitrate | $NO_3^-$ | none |
| Nitrite | $NO_2^-$ | none |
| Chloride | $Cl^-$ | 151.000 |
| Bromide | $Br^-$ | 13.000 |
| Iodide | $I^-$ | 2.880 |
| Fluoride | $F^-$ | 2.700 |
| Sulfate | $SO_4^{2-}$ | 4.080 |
| Hydrocarbonate | $HCO_3^-$ | 83.000 |
| Sulfide | $S^{2-}$ | none |
| Total phosphate | $PO_4^{3-}$ | none |
| Metaboric acid | $HBO_2$ | 19.130 |
| Metasilicic acid | $H_2SiO_3$ | 0.420 |
| Carbon dioxide | $CO_2$ | none |
| Dissolved oxygen | $O_2$ | none |

Table II

| The parameters of flue-ash formed in power stations used in the process | | |
|---|---|---|
| | | % by weight |
| Chemical composition | $SiO_2$ | 45-60 |
| | $Al_2O_3$ | 16-24 |
| | $Fe_2O_3$ | 8-16 |
| | $CaO$ | 1-6 |
| | $MgO$ | 1-6 |
| | C (coke) | 5-8 |
| Particle size | 95 % under 45 $\mu$m | |
| | 75% under 32 $\mu$m | |

The suspension is left to stand for 3 days then the physically bound water is removed (by filtration and drying), the remaining air-dry (moisture content is below 5 %) sample is powdered, then mixed with such an amount of water (comprising no therapeutically active ingredient) having a temperature of 50-60 °C which enables the setting of the moisture content of the medical mud prepared from flue-ash formed in power stations in accordance with the intended use. The results of the examinations carried out with medicinal muds prepared from flue-ash formed in power stations, having a moisture content of 33, 40 and 50 %, respectively are summarized in Table III.

Table III

| The amount of water-soluble components expressed in ions | | | |
|---|---|---|---|
| Component | Composition of peloid suitable for direct use calculated for 1000 g of the contained water (g) | | |
| | 33 % | 40 % | 50 % |
| | | moisture content | |
| potassium | 8.34 | 6.86 | 5.5 |
| sodium | 10.3 | 8.60 | 6.9 |
| lithium | 0.30 | 0.25 | 0.20 |
| chloride | 15.55 | 12.81 | 10.15 |
| bromide | 1.33 | 1.09 | 0.86 |
| iodide | 0.29 | 0.24 | 0.19 |
| fluoride | 0.28 | 0.23 | 0.19 |
| hydrocarbonate | 8.47 | 6.99 | 5.64 |
| metaboric acid | 1.97 | 1.62 | 1.30 |
| metasilicic acid | 0.043 | 0.035 | 0.028 |

As it is shown by the above table, in the peloid suitable for direct use the concentration of the active ingredients is about 10.3, 8.5 and 6.8% of the active ingredients being in the mother liquor, respectively, depending on the moisture content of the peloid. Thus the adsorption/desorption effectiveness of the flue-ash used for preparing the medicinal mud prepared from flue-ash formed in power stations varies within the range of 6.8 - 10.3 % calculated for 1000 g of the peloid, depending on the moisture content of the peloid suitable for direct use.

As a comparison we show the composition of the medicinal water of Sárvár (Liquid A) and that of the bath prepared according to prescriptions from the commercially available medicinal salt of Sárvár by dissolving 15 g of salt in one litre of water (liquid B).

Table IV

| The ion composition of one liter of water (g/l) | | |
|---|---|---|
| Component | Liquid A | Liquid B |
| potassium | 0.91 | 0.27 |
| sodium | 15.89 | 5.55 |
| lithium | 0.016 | 0.001 |
| chloride | 22.70 | 8.85 |
| bromide | 0.10 | 0.019 |
| iodide | 0.016 | 0.0018 |
| fluoride | 0.004 | 0.0008 |
| hydrocarbonate | 4.45 | 0.069 |
| metaboric acid | 0.27 | 0.0113 |
| metasilicic acid | - | 0.018 |

Comparing the data of Tables III and IV it can be established that the medicinal mud prepared from flue-ash formed in power stations (having any moisture content) comprises the therapeutically active iodide, bromide, fluoride ions, metaboric acid, metasilicic acid, potassium and hydrocarbonate ions in significantly higher concentration than the original medicinal water of Sárvár or the solution prepared from the bath-salt of Sárvár. Simultaneously, the sodium chloride content of the mud is less than that of the medicinal water of Sárvár, therefore the skin irritation is smaller.

Example 2

The process of Example 1 is followed for the preparation of the medicinal mud of the invention except that 200 g of fine flue-ash are added to 100 g of the mother liquor. Then a thick flue-ash mud is obtained which is subjected to drying in order to eliminate the physically bound water. Further on the air-dry mud is treated according to Example 1. Using this mixing ratio the adsorption/desorption effectivity (taking into consideration all of the active ingredients and calculating the average) varies within 6.5 - 10.1 % depending on the moisture content (33 - 50 %).

Example 3

The process of Example 1 is followed for the preparation of the medicinal mud of the invention except that a fraction of flue-ash formed in power stations having higher particle size, but comprising abundant small pores and inner spaces is used. The parameters of this fraction are listed in Table V.

Table V

| The parameters of the flue-ash used | | |
|---|---|---|
| Chemical composition | $SiO_2$ | 35-65 % |
| | $Al_2O_3$ | 10 - 30 % |
| | $Fe_2O_3$ | 3-10 % |
| | CaO | 0.6-9 % |
| | MgO | 1-2 % |
| | C (coke) | 15-35 % |
| Particle size | 4 % | under 100 $\mu$m |
| | 15 % | 100-200 $\mu$m |
| | 23 % | 200- 400 $\mu$m |
| | 54 % | 400-800 $\mu$m |
| | 4 % | above 800 $\mu$m |

Using this kind of flue-ash fraction the adsorption/desorption effectivity is 5.8-8.7 % in the case of a peloid suitable for direct use, having a moisture content of 33-50 %.

Example 4

The process of Example 1 is followed except that the porous flue-ash according to Example 3 is used and 50 g of this flue-ash are added to 100 g of mother liquor. Thus a thin, liquid-like mud is obtained from which the water is removed by decantation and drying. Further on the procedure of Example 1 is followed. Using the above mixing ratio the adsorption/desorption effectivity is 5.9-8.7 % if the medicinal mud has a moisture content of 33-50 %.

Example 5

The process of Example 1 is followed except that instead of the mother liquor of the medicinal water of Sárvár, the original medicinal water (the composition of which is shown in Table IV) is used for the preparation of medicinal mud. Though the original medicinal water comprises the active ingredients in much lower concentration than the mother liquor, the adsorption/desorption effectivity of flue-ash added to the medicinal water is the same as in Example 1, i.e. 6.1-9.2 % when the moisture content of the mud is 33-50 %.

Example 6

The process of Example 5 is followed except that the porous flue-ash fraction of Table V is used instead of fine flue-ash. The adsorption/desorption effectivity of the porous flue-ash is 5.2-7.8 % depending on the moisture content (33-50 %).

Example 7

The process of Example 1 is followed except that a 15 g/l solution of the bath-salt of Sárvár (the composition of which is shown in Table IV) is used instead of the mother liquor of the medicinal water of Sárvár. The adsorption/desorption effectivity of the fine flue-ash is 6.0-9.1 %.

Example 8

The process of Example 7 is followed except that the porous flue-ash fraction (Table V) is used instead of fine flue-ash. The measured adsorption/desorption effectivity is 5.1-7.6%.

Example 9

The process of Example 1 is followed except that the medicinal water of Hévíz (the composition thereof is shown in Table VI) is used instead of the mother liquor of Sárvár. The adsorption/desorption effectivity is 6.0-9.0 %.

Table VI

The composition of medicinal water of Hévíz

(the amount of the components is given in mg/l)

| Component | | |
|---|---|---|
| Potassium | $K^+$ | 6.52 |
| Sodium | $Na^+$ | 14.67 |
| Ammonium | $NH_4^+$ | undetectable |
| Calcium | $Ca^{2+}$ | 37.73 |
| Magnesium | $Mg^{2+}$ | 337.40 |
| Iron | $Fe^{2+}$ | undetectable |
| Manganese | $Mn^{2+}$ | undetectable |

total amount of cations: 176.32

7

| Nitrate | $NO_3^-$ | undetectable |
|---|---|---|
| Nitrite | $NO_2^-$ | undetectable |
| Chloride | $Cl^-$ | 25.000 |
| Bromide | $Br^-$ | undetectable |
| Iodide | $I^-$ | undetectable |
| Fluoride | $F^-$ | ·1.4 |
| Sulfate | $SO_4^{2-}$ | 94.62 |
| Hydrocarbonate | $HCO_3^-$ | 414.87 |
| Sulfide | $S^{2-}$ | 0.069 |

total amount of anions: 5355.95

| Metaboric acid | $HBO_2$ | 4.71 |
|---|---|---|
| Metasilicic acid | $H_2SiO_3$ | 14.56 |
| Carbon dioxide | $CO_2$ | 60.20 |
| Dissolved oxygen | $O_2$ | 0.36 |

total: 792.10

## Example 10

The process of Example 9 is followed except that the porous flue-ash fraction of Table V is used instead of fine flue-ash. The adsorption/desorption effectivity is 5.1-7.7 %.

## Example 11

The process of Example 1 is followed except that the medicinal water of Hajdúszoboszló (the composition of which is shown in Table VII) comprising iodine is used instead of the mother liquor of Sárvár.

Table VII

| The composition of the medicinal water of Hajdúszoboszló (the amount of the ingredients is given in mg/l) | | |
|---|---|---|
| Potassium | $K^+$ | 17.46 |
| Sodium | $Na^+$ | 2010.6 |
| Ammonium | $NH_4^+$ | 1.02 |
| Calcium | $Ca^{2+}$ | 9.053 |
| Magnesium | $Mg^{2+}$ | 7.28 |
| Iron | $Fe^{2+}$ | trace |
| Aluminium | $Al^{3+}$ | trace |
| Nitrate | $NO^{3-}$ | undetectable |
| Nitrite | $NO^-$ | undetectable |
| Chloride | $Cl^-$ | 2305.8 |
| Bromide | $Br^-$ | 12.38 |
| Iodide | $I^-$ | 7.79 |
| Fluoride | $F^-$ | 1.44 |
| Sulfate | $SO_4^{2-}$ | 5.00 |
| Hydrocarbonate | $HCO_3^-$ | 1390.0 |
| Carbonate | $CO_3^{2-}$ | 23.20 |
| Metaboric acid | $HBO_2$ | 109.6 |
| Metasilicic acid | $H_2SiO_3$ | 40.18 |
| Carbon dioxide | $CO_2$ | undetectable |
| Dissolved oxygen | $O_2$ | undetectable |

The adsorption/desorption factor is 6.3-9.5.

Example 12

The process of Example 11 is followed except that the porous flue-ash fraction (Table V) is used instead of fine flue-ash. The adsorption/desorption effectivity is 5.3-8.0 %.

Example 13

Medicinal mud of the invention is prepared for direct use. To 100 g of the mother liquor formed in course of distillation of the medicinal water of Sárvár 100 g of fine flue-ash (Table II) are added. The suspension (mud) thus obtained is left to stand for 3 days, then it is directly used by slight warming (40-60 °C) the mud in order to enhance the desorption of the active ingredients. The adsorption/desorption effectivity of medicinal mud prepared from flue-ash formed in power stations (taking into consideration all active ingredients and calculating the average value) is 91 %.

Example 14

The process of Example 13 is followed except that 200 g of fine flue-ash are added to 100 g of the mother liquor. Thus a thick mass of mud is obtained. The adsorption/desorption effectivity is 87 %.

Example 15

The process of Example 13 is followed except that 25 g of fine flue-ash are added to 100 g of mother liquor, thus a thick liquid mud is obtained. The adsorption/desorption effectivity is 95 %.

9

Example 16

The process of Example 13 is followed except that the porous flue-ash fraction is used instead of fine flue-ash. The adsorption/desorption effectivity is 89 %.

Example 17

The process of Example 13 is followed except that the original medicinal water of Sárvár is used instead of the mother liquor. The adsorption/desorption effectivity is 90 %.

Example 18

The process of Example 13 is followed except that instead of the mother liquor of the medicinal water of Sárvár, a 15 g/l solution of the bath-salt of Sárvár (Table IV) is used. The adsorption/desorption effectivity is 90 %.

Example 19

The process of Example 13 is followed except that instead of the mother liquor of Sárvár the medicinal water of Héviz (the composition thereof is shown in Table VI) is used. The adsorption/desorption effectivity is 91 %.

Example 20

The process of Example 13 is followed except that the medicinal water of Hajdúszoboszló comprising iodine (the composition thereof is shown in Table VII) is used instead of the mother liquor of Sárvár. The adsorption/desorption effectivity is 89 %.

Example 21

The process of any of Example 17-20 is followed except that instead of fine flue-ash the porous flue-ash fraction (Table V) is used. The adsorption/desorption effectivity is almost the same with some percentile difference.

Example 22

In the following Tables the composition of the natural peloids of the lake Héviz and Velence as well as the river Maros is given.

A) Peloid of Héviz

Moisture content of the original mud: 84.5 %.
Moisture content of the commercially available air-dry sample: 4.3 %.
Moisture content of the sample suitable for use: 42.5 %.

Table VIII

| Water-soluble inorganc composition (expressed as ions) of the peloid calculated for 1000 g of the contained water (g) | | |
|---|---|---|
| Component | | |
| Sodium | $Na^+$ | 0.433 |
| Calcium | $Ca^{2+}$ | 3.572 |
| Magnesium | $Mg^{2+}$ | 0.744 |
| Sulfate | $SO_4^{2-}$ | 7.591 |
| Hydrocarbonate | $HCO_3^-$ | 5.353 |
| Metasilicic acid | $H_2SiO_3$ | 0.068 |
| Elements occuring in smaller amounts: Fe, K, Cl, I. | | |

B) Peloid of Velence

Moisture content of the original mud: 80.91 %.
Moisture content of the peloid suitable for use: 78.2 %.

Table IX

| Water-soluble inorganic composition (expressed in ions) of the peloid calculated for 1000 g of the contained water (g) | | |
|---|---|---|
| Component | | |
| Potassium | $K^+$ | 0.142 |
| Sodium | $Na^+$ | 0.494 |
| Calcium | $Ca^{2+}$ | 0.135 |
| Magnesium | $Mg^{2+}$ | 0.231 |
| Chloride | $Cl^-$ | 0.260 |
| Sulfate | $SO_4^{2-}$ | 0.196 |
| Hydrocarbonate | $HCO_3^-$ | 2.408 |
| Metasilicic acid | $H_2SiO_3$ | 0.179 |
| Hydrogen sulfide | $H_2S$ | 0.050 |

C) Peloid of Maros

Moisture content of the original mud: 36.37 %.
Moisture content of the peloid suitable for use: 48.70 %.

11

Table X

| Water-soluble inorganic composition (expressed in ions) of the peloid calculated for 1000 g of the contained water (g) | | |
|---|---|---|
| Component | | |
| Potassium | $K^+$ | 0.041 |
| Sodium | $Na^+$ | 0.105 |
| Calcium | $Ca^{2+}$ | 1.433 |
| Magnesium | $Mg^{2+}$ | 0.357 |
| Iron | $Fe^{2+}$ | 0.021 |
| Ammonium | $NH_4^+$ | 0.034 |
| Manganese | $Mn^{2+}$ | 0.053 |
| Chloride | $Cl^-$ | 0.047 |
| Hydrocarbonate | $HCO_3^-$ | 4.497 |
| Sulfate | $SO_4^{2-}$ | 0.195 |
| Silicate | $SiO_4^{2-}$ | 0.686 |

Comparing the natural peloids and that prepared according to the invention, it can be seen that the medicinal mud prepared from mother liquor of Sárvár and flue-ash formed in power stations contains the most of potassium, sodium and hydrocarbonate ions. Furthermore, iodide, fluoride and bromide, which are important from therapeutic viewpoint and are absent from the natural peloids presented hereinabove, are contained in a high concentration, further it can be seen that the particle size distribution of the fine flue-ash fraction used in the process of the invention (see Table II) is the same as Maros mud which is regarded as the best one of the natural peloids from this viewpoint. The particle size distribution of Maros mud: 96 % under 50 $\mu$m, 67,7 % under 20 $\mu$m, respectively.

## Claims

1. A process for the preparation of artificial medicinal mud which comprises suspending 25-200 % by weight of flue-ash (calculated for medicinal water or concentrated medicinal water) formed in power stations, having a particle size of 0.002-1.00 mm, a CaO content of less than 10 % of CaO and a carbon (coke) content of 5-25 %, in a medicinal water or concentrated medicinal water and leaving the suspension to stand for 24-72 hours before use.

2. A process as claimed in Claim 1 characterized by using 100 % of flue-ash formed in power stations calculated for the weight of the medicinal water.

3. A process as claimed in Claim 1 further characterized by removing the physically bound water and forming an air-dry product.

4. A process as claimed in Claim 3 characterized by producing a product having a moisture content of less than 5 %.

5. A process as claimed in Claim 3 further characterized by setting the moisture content of the air-dry product when used to 33-50 %.

6. A process as claimed in any of Claims 1 or 5 further characterized by heating the medicinal mud thus obtained to a temperature of 40-60 °C when used.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 705 433  (V. BERTHELSEN)<br>* Claims 1-3; page 10, last paragraph; page 11, paragraph 1 *<br>--- | 1-3 | A 61 K   35/08<br>A 61 K    7/48 //<br>(A 61 K   35/08<br>A 61 K   35:02 ) |
| X | EP-A-0 107 751  (C. KOMAKINE)<br>* Claims 1,3 *<br>----- | 1,2 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 27-06-1989 | PEETERS J.C. |

EPO FORM 1503 03.82 (P0401)